# EUROPÄISCHE PATENTSCHRIFT

(11) **EP 2 873 427 B1**
(45) Veröffentlichungstag und Bekanntmachung des Hinweises auf die Patenterteilung: **04.01.2017**
(21) Anmeldenummer: 13005347.3
(22) Anmeldetag: 13.11.2013
(51) Int. Cl.: A61L 9/12

(54) **Raumbeduftungsgerät**
Air-freshener device
Appareil de parfum d'ambiance

(43) Veröffentlichungstag der Anmeldung: 20.05.2015
(73) Patentinhaber: Trisa Electronics AG, 6234 Triengen (CH)
(72) Erfinder: Studer, Philipp, CH-6234 Triengen (CH)
(74) Vertreter: Schaad, Balass, Menzl & Partner AG

(56) Entgegenhaltungen:
- WO-A1-2006/074562
- WO-A1-2011/106889
- WO-A1-2013/012442
- WO-A2-2012/101642
- US-A1- 2004 241 053
- US-A1- 2006 196 100
- US-A1- 2010 243 754
- US-A1- 2011 198 409
- US-B1- 6 197 263

## Beschreibung

Die vorliegende Erfindung betrifft ein Raumbeduftungsgerät gemäss Anspruch 1, eine auswechselbare Duftkapsel gemäss Anspruch 11, sowie ein Set gemäss Anspruch 17.

Ein Raumbeduftungsgerät und ein Verfahren zum Beduften von Räumen ist aus dem Dokument WO96/31741 bekannt. Das Raumbeduftungsgerät weist einen Luftleitkanal, ein Gebläse zum Fördern von zu beduftender Luft durch den Luftleitkanal, einen Vorratsbehälter für einen Duftstoff beinhaltenden Flüssigkeitsvorrat und eine Flüssigkeitverdunstungsfläche auf, über welche der zu beduftende Luftstrom zur Aufnahme von verdunstendem Luftstoff geleitet wird. Um die Duftstoffaufnahme durch den Luftstrom gut steuerbar zu gestalten, weist der Vorratsbehälter eine nach unten weisende Entleerungsöffnung auf, durch welche die Flüssigkeit den Vorratsbehälter nach unten verlässt um sich auf eine unter der Entleerungsöffnung gelegene, bezüglich der Entleerungsöffnung schräg nach unten geneigte, als Flüssigkeitsverdunstungsfläche dienende Oberfläche eines Verdunstungskörpers zu verteilen. Es ist ein die Entleerungsöffnung beherrschendes Dosierventil zur dosierten Abgabe von Flüssigkeit aus dem Vorrat an die Schrägfläche vorgesehen. Die Schrägfläche ist innerhalb des Luftleitkanals derart angeordnet, dass der zu beduftende Luftstrom intensiv über die Schrägfläche streicht, sodass dabei am unteren Ende der Schrägfläche praktisch keine Flüssigkeit mehr anfällt oder überschüssig ist.

Die US 2011/198409 offenbart eine Kartusche mit einer auswechselbaren Beduftungskapsel, Luftaustrittsöffnungen und einer Heizung, welche die Emission gasförmiger Duftstoffe aus der Kapsel fördert. Die Kartusche kann in einer Kammer eines baumförmigen Beduftungsgeräts oberhalb eines Lüfters angeordnet sein, wobei der Lüfter Luft durch das baumförmige Beduftungsgerät bläst, so dass einerseits die Heizung gekühlt und andererseits Luft durch die Kartusche strömt, um die Duftstoffe zu verteilen.

Die WO 2013/012442 offenbart ein Lufterfrischungsgerät in Form eines USB-Sticks, welcher am freien Ende einen Aufnahmebehälter aufweist, in welchen eine auswechselbare Duftkapsel eingesetzt werden kann. Das Lufterfrischungsgerät kann über einen USB-Anschluss mit Strom versorgt werden und umfasst ein Heizelement, welches unterhalb der Duftkapsel angeordnet ist. Durch Erwärmen eines in der Duftkapsel enthaltenen Duftspeicherpads werden im Pad enthaltene Duftstoffe via Emission durch Öffnungen in der Duftkapsel an die Umgebung abgegeben.

Die WO 2012/101642 offenbart ein Beduftungsgerät umfassend eine Duftkapsel mit einem Zerstäuber. Die Duftkapsel enthält mehrere Duftreservoirs, wobei mittels eines ein piezoelektrisches Element umfassenden Vibrationsmechanismus eine bestimmte Menge an Duftstoff aus einem jeweiligen Duftreservoir abgegeben und mittels Zerstäuber verteilt werden kann.

Die US 6,197,263 betrifft einen Lufterfrischer zur Verwendung in einem Auto, der einen Stromanschluss sowie eine Duftabgabeeinheit mit einem Heizelement und einer Aufnahme für auswechselbare Gel-Duftkapseln umfasst. Durch Aufwärmen der Gel-Duftkapseln mittels Heizelement wird die Abgabe von Duftstoffen gefördert. Die Duftabgabeeinheit kann an der Luftaustrittsöffnung der Lüftung des Autos angeschlossen werden, so dass Luft aus der Lüftung durch Öffnungen in Duftabgabeeinheit, über die Oberfläche der Gel-Duftkapseln strömt und die mit Duftstoff beladene Luft beim anschliessenden Austritt aus der Duftabgabeeinheit via Öffnungen den Duftstoff im Auto verteilt.

Es ist eine Aufgabe der vorliegenden Erfindung, ein Raumbeduftungsgerät zu schaffen, welches ein sauberes und einfaches Handling ohne Wartungsaufwand ermöglicht. Weiter ist es eine Aufgabe der vorliegenden Erfindung eine Beduftungskapsel für das Raumbeduftungsgerät sowie ein Set vorzuschlagen.

Diese Aufgabe wird mit einem Raumbeduftungsgerät gemäss Anspruch 1, einer Beduftungskapsel gemäss Anspruch 11 und einem Set gemäss Anspruch 17 gelöst.

Das erfindungsgemässe Raumbeduftungsgerät weist ein Gehäuse mit einem Lufteinlass, einem Luftauslass und einer Einführöffnung auf. Im Innenraum des Gehäuses verläuft ein Luftkanal vom Lufteinlass zum Luftauslass. Ein Ventilator fördert im Betrieb vom zum beduftenden Raum durch den Lufteinlass angesaugte Luft durch den Luftkanal zum Luftauslass, wo die beduftete Luft in den Raum ausgeblasen wird.

Das Gehäuse weist weiter eine Einführöffnung auf und im Gehäuse ist ein, vorzugsweise mit der Einführöffnung fluchtendes Aufnahmefach angeordnet, in welches durch die Einführöffnung eine auswechselbare Beduftungskapsel in das Aufnahmefach in eine Arbeitsstellung hineinbewegbar ist. Die Beduftungskapsel ist auch wieder aus dem Aufnahmefach heraus bewegbar, vorzugsweise ebefalls durch die Einführöffnung. Es ist jedoch auch möglich, dass die (verbrauchte) Beduftungskapsel durch eine separate Öffnung im Gehäuse aus dem Aufnahmefach herausbewegbar ist oder dass die Beduftungskapsel aus dem Aufnahmefach in einen Auffangbehälter herausbewegbar ist. Letzerer ist bevorzugt im Gehäuse und diesem entnehmbar angeordnet.

Die Einführöffnung ist bevorzugt an eine Rückseite.des Gehäuses angeordnet und der Lufteinlass und der Luftauslass befinden sich bevorzugt in einem unteren beziehungsweise oberen Rückbereichsbereich des Gehäuses.

Die Beduftungskapsel weist einen Lufteintritt, einen Duftstoffträger sowie einen Luftaustritt auf. Da der Luftkanal durch das Aufnahmefach hindurch verläuft, wird die aus dem Raum angesaugte Luft durch die Beduftungskapsel hindurchgefördert, wobei der Duftstoffträger Duftstoff an die Luft abgibt, welche als beduftete Luft durch den Luftauslass in den Raum ausgeblasen wird.

Die Verwendung von auswechselbaren Beduftungskapseln ermöglicht ein angenehmes Raumduft-Klima mit individuellen Düften zu schaffen. Dazu werden auswechselbare Beduftungskapseln mit den entsprechenden Düften zur Verfügung gestellt.

Es ist ein sauberes und einfaches Handling ohne Wartungsaufwand gewährleistet, da das "Nachfüllen" des Raumbeduftungsgeräts einzig durch das Auswechseln der Beduftungskapsel erfolgt. Ein Hantieren mit Flüssigkeiten, insbesondere das Überschwappen oder Ausschütten der Flüssigkeit oder Ausfliessen von Flüssigkeit in das Raumbeduftungsgerät selber, ist vermieden.

Da die Beduftung der Luft einzig durch das Durchströmen durch die Beduftungskapsel erfolgt und der Duftstoff nicht erwärmt wird, kann auf Heizelemente oder Ultraschalltechnologien verzichtet werden, was ungewollte, gegebenenfalls gesundheitschädliche chemische Reaktionen und das Zersetzen von Duftstoff vermeidet.

Bevorzugt weist das Gehäuse eine einzige Einführöffnung auf, durch welche hindurch die Duftkapsel in einer Einschieberichtung in das Aufnahmefach hineinschiebbar ist. Durch diese Einführöffnung ist die Duftkapsel entgegen der Einschieberichtung aus dem Aufnahmefach wieder herausschiebbar oder herausziehbar. Dies ermöglicht einen sehr einfachen Aufbau des Raumbeduftungsgeräts.

Bevorzugt ist dem Aufnahmefach ein Schloss zugeordnet, welches die in das Aufnahmefach hineingeschobene Beduftungskapsel in Arbeitsstellung fixiert. Bevorzugt ist das Schloss derart ausgebildet, dass es nach erneuter Druckausübung in Einschieberichtung auf die Druckkapsel diese wieder frei gibt, insbesondere entgegen der Einschieberichtung aus der Arbeitsstellung bewegt. Dies vereinfacht weiter das Handling des Raumbeduftungsgeräts. Derartige Schlösser - sogenannte Schnappverschlüsse oder "draw locker" - sind bekannt.

Bevorzugt fluchtet die in Arbeitsstellung sich befindende Duftkapsel wenigstens annähernd mit der Aussenfläche des Gehäuses. Dies verhindert das ungewollte Herausbewegen der Beduftungskapsel aus der Arbeitsstellung und ermöglicht ein gefälliges Design.

Zum Zusammenwirken mit dem Schloss kann die Beduftungskapsel eine entsprechende vorstehende Rastnase oder eine Rastausnehmung aufweisen.

Bevorzugt verschliesst die Duftkapsel in Arbeitsstellung die Einführöffnung wenigstens annähernd vollständig. Damit kann auf separate Dichtungselemente verzichtet werden.

Die Beduftungskapsel weist vorzugsweise ein Kapselgehäuse mit einer ersten Wandseite und einer zweiten Wandseite auf. Bevorzugt liegen die erste Wandseite und die zweite Wandseite einander gegenüber. Die erste Wandseite bildet den Lufteintritt, welcher bevorzugt durch eine Vielzahl in einem Lochfeld angeordnete Durchströmungsöffnungen gebildet ist. Entsprechend bildet die zweite Wandseite den Luftaustritt, welcher bevorzugt durch eine Vielzahl in einem weiteren Lochfeld angeordnete weitere Durchströmöffnungen gebildet ist. Im Innern des Kapselgehäuses ist der Duftstoffträger angeordnet.

Bevorzugt sind die erste Wandseite und die zweite Wandseite durch einen in sich geschlossenen Mantel miteinander verbunden.

Die Duftkapsel kann die Form eines Kreiszylinders oder Elypsenzylinders aufweisen. Bevorzugt ist das Kapselgehäuse jedoch wenigstens annähernd quaderförmig ausgebildet.

Bevorzugt ist die Höhe der Beduftungskapsel kleiner als die Abmessungen in der Aufsicht gesehen. Bei einer wenigstens annähernd quaderförmigen Ausführungsform des Kapselgehäuses, sind die Länge sowie die Breite der ersten und zweiten Wandseite grösser, vorzugsweise wenigstens dreimal so gross wie die Höhe der diese Wandseiten dichtend miteinander verbindenden Mantel des Kapselgehäuses.

Der Duftstoffträger ist bevorzugt porös, insbesondere filzartig ausgebildet. Dies ermöglicht die sichere tropffreie Aufnahme des Duftstoffs. Überdies ist eine gleichmässige Beduftung der Luft über eine lange Zeit, beispielsweise einen Monat, gewährleistet.

Bevorzugt weist der Duftstoffträger einen mit dem Duftstoff und vorzugsweise mit einem Neutralisationsstoff getränkten Duftträgerbereich auf. Um diesen herum verläuft bevorzugt ein duftstofffreier Randbereich, welcher bevorzugt poröser ausgebildet ist als der Duftträgerbereich. Durch die unterschiedlichen Porösitäten kann einerseits der Widerstand gegen die strömende Luft reduziert und andererseits ein gleichmässiges Beduften der Luft über lange Zeit ermöglicht werden.

Ein besonders einfacher Aufbau des Raumbeduftungsgeräts ist dadurch möglich, dass das Gehäuse mantelseitig aus einem Abschnitt eines - bevorzugt in Umfangsrichtung in sich geschlossenen - gezogenen Hohlprofils, vorzugsweise aus Metall insbesondere aus Aluminium gefertigt ist. Das Gehäuse kann bodenseitig am Mantelteil durch eine befestigte Bodenplatte verschlossen sein, welche bevorzugt eine Printplatte mit der elektronischen Schaltung zur Steuerung und Speisung des Ventilators trägt. Weiter weist das Gehäuse bevorzugt einen Gehäusedeckel auf, welcher am Mantelteil befestigt ist. Dieser Deckel kann beispielsweise aus Lochblech gefertigt sein um vollständig oder teilweise als Luftauslass zu dienen.

Bevorzugt erstreckt sich der Mantelteil über die gesamte Höhe des Gehäuses und bevorzugt sind die Bodenplatte und der Deckel im Mantelteil versenkt angeordnet.

Die Einführöffnung ist bei dieser Ausführungsform in einer durch den Abschnitt gebildeten Seitenfläche - bevorzugt in einer Rückseite des Gehäuses ausgebildet.

Bevorzugt ist das Aufnahmefach zusammen mit dem Ventilator als Baueinheit ausgebildet, welche in Längsrichtung des Mantelteils in diesen hineingeschoben ist und am Abschnitt des Hohlprofils befestigt ist, wobei das Aufnahmefach mit der Einfuhröffnung fluchtet.

Damit kann auf einfache Art und Weise sichergestellt werden, dass die durch Ventilation geförderte Luft durch die Beduftungskapsel strömt.

Die elektronische Steuerung weist bevorzugt einen Schalter zum Ein- und Ausschalten des Ventilators sowie bevorzugt zum Einstellen von gewünschten, insbesondere vorbestimmten Drehzahlen des Ventilators auf. Weiter kann die elektronische Schaltung eine Signallampe - oder Signallampen - aufweisen, welche den Betriebszustand des Raumbeduftungsgeräts anzeigt, bevorzugt auch angibt, wann die Beduftungskapsel ausgewechselt werden sollte. Dies kann beispielsweise durch das Aufaddieren der Einschaltzeiten, vorzugsweise unter Berücksichtigung der gewählten Drehzahl des Ventilators, ermöglicht werden. Sobald die vorgegebene Benutzungsdauer einer Beduftungskapsel erreicht ist, wird das entsprechende Signal erzeugt. Es kann ein Mittel, beispielsweise ein Mikroschalter, vorgesehen sein, welcher an die elektronische Schaltung jeweils ein Signal abgibt, wenn eine Beduftungskapsel ausgewechselt wird, wodurch das Additionselement der elektronischen Schaltung zum Addieren der Einschaltzeiten zurückgesetzt wird. Bevorzugt leuchtet die Signallampe beziehungsweise leuchten die Signallampen auf der Vorderseite des Gehäuses.

Die Beduftungskapseln sind bevorzugt von einer Lieferverpackung luftdicht umgeben. Vor dem Einführen der Beduftungskapsel in das Raumbeduftungsgerät, wird sie der Lieferverpackung entnommen und dann durch die Einführungsöffnung in das Aufnahmefach hineingeführt.

Das Aufnahmefach ist bevorzugt auf die Aussenform der Beduftungskapsel derart abgestimmt, dass, bevorzugt ohne das Notwendigmachen von speziellen Dichtungsmittel, die Luft durch die Beduftungskapsel hindurch gezwungen wird.

Unterschiedliche Beduftungsstoffe und Neutralisationsstoffe sind bekannt, beispielsweise werden solche unter der Marke "airomat" von market-thing gmbh, CH-6232 Greuensee, geliefert.

Die vorliegende Erfindung wird anhand eines in der Zeichnung dargestellten Ausführungsbeispiels näher erläutert. Es zeigen rein schematisch:
- Fig. 1: in perspektivischer Darstellung ein Raumbeduftungsgerät wobei das Gehäuse mantelseitig zur besseren Erkennung der in das Gehäuse eingebauten Komponenten durchsichtig dargestellt ist;
- Fig. 2: in Explosionsdarstellung das Raumbeduftungsgerät, gemäss Fig.1;
- Fig. 3: in perspektivischer Ansicht eine auswechselbare Beduftungskapsel;
- Fig. 4: in Ansicht die Vorderseite der Beduftungskapsel, gemäss Fig. 3;
- Fig. 5: die Beduftungskapsel gemäss Fig. 3 und 4 in der Aufsicht;
- Fig. 6: Die Beduftungskapsel in einem Schnitt entlang der Linie VI-VI der Fig. 5;
- Fig. 7: in Aufsicht ein im Innern des Kapselgehäuses der Beduftungskapsel, gemäss den Fig. 3 bis 6 angeordneter Duftstoffträger; und

Fig. 8 den Duftstoffträger gemäss Fig. 7 in einem Schnitt entlang der Linie VIII-VIII der Fig. 7.

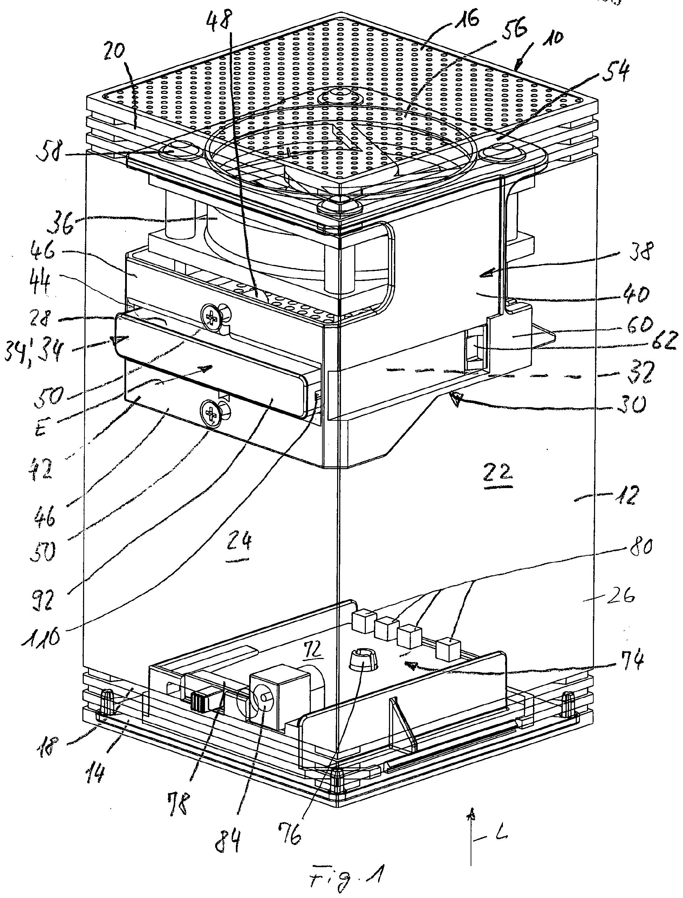

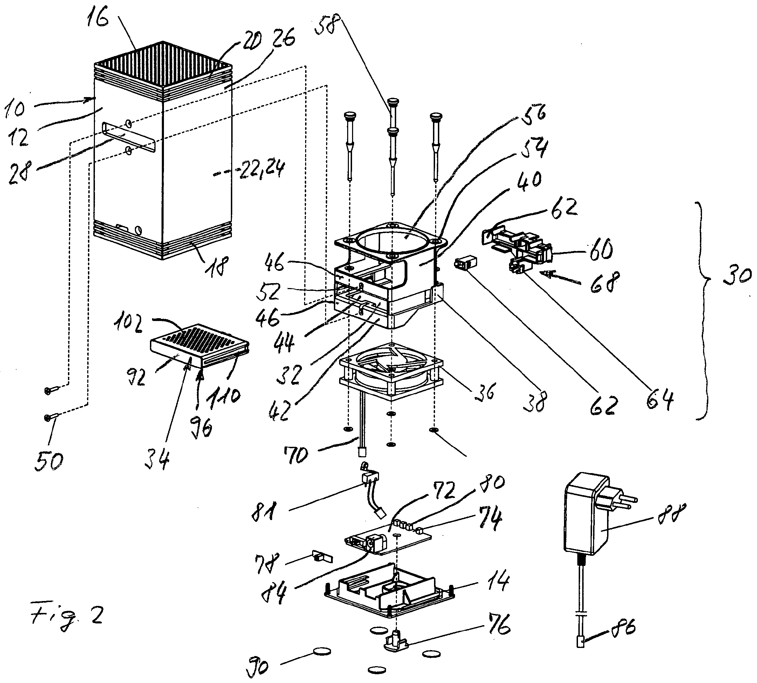

Bei der in den Figuren 1 und 2 gezeigten Ausführungsform eines Raumbeduftungsgeräts besteht dessen Gehäuse 10 aus einem Mantelteil 12, einer Bodenplatte 14 und einer Deckelplatte 16.

In einem unteren Endbereich weist das Gehäuse 10, im gezeigten Ausführungsbeispiel der Mantelteil 12, einen Lufteinlass 18 auf. Im oberen Endbereich ist das Gehäuse 10, hier der Mantelteil 12 und die Deckelplatte 16, mit einem Luftauslass 20 versehen.

Der vom Gehäuse 10 begrenzte Innenraum 22 bildet einen Luftkanal 24, welcher vom Lufteinlass 18 zum Luftauslass 20 führt.

Im gezeigten Ausführungsbeispiel ist der Lufteinlass 18 durch in Umfangsrichtung verlaufende Schlitze im Mantelteil 12 gebildet. Der Luftauslass 20 ist ebenfalls durch entsprechende Schlitze im Mantelteil 12 sowie durch die als Lochplatte ausgebildete Deckelplatte 16 realisiert.

Der Mantelteil 12 des gezeigten Ausführungsbeispiels ist aus einem Abschnitt 26 eines in Umfangsrichtung in sich geschlossenen Aluminiumprofils gefertigt. Dieses weist einen quadratischen Aussenquerschnitt und einen zu diesem konzentrischen quadratischen Innenquerschnitt auf, wobei die Eckbereiche des Innenquerschnitts abgeschrägt ausgebildet sind, um die Kantenbereiche des Profils zu verstärken. Diese Ausführungsform hat im gezeigten Beispiel den Vorteil, dass die den Lufteinlass 18 und den Luftauslass 18 im Mantelteil 12 bildenden Schlitze in sich geschlossen umlaufend ausgebildet werden können wobei in den Kantenbereichen, infolge der Abschrägung des Innenquerschnitts, Profilteile 26 verbleiben und ansonsten die Schlitze durch die Wand des Mantelteils 12 durchgehend sind.

Das Gehäuse 10, im Ausführungsbeispiel der Mantelteil 12, weist auf seiner Rückseite eine hier annähernd rechteckförmige Einführöffnung 28 auf. Diese ist zwischen dem Lufteinlass 18 und dem Luftauslass 20 angeordnet.

Im Innenraum 22 des Gehäuses 10 ist eine Baueinheit 30 befestigt, welche einerseits ein Aufnahmefach 32 für auswechselbare Beduftungskapseln 34 bildet und andererseits einen Ventilator 36 aufweist, welcher dazu bestimmt ist, im Betrieb Luft aus dem Raum, in welchem das Raumbeduftungsgerät steht, beispielsweise einem Wohnraum, einem Schlafzimmer, einer Toilette oder einem Büroraum, durch den Lufteinlass 18 anzusaugen und durch den Luftkanal 24 zum Luftauslass 20 zu fördern wo die beduftete Luft wieder in den Raum abgegeben wird. Zum Beduften der Luft verläuft der Luftkanal 24 durch das Aufnahmefach 32, wodurch die Luft gezwungen wird, die Beduftungskapsel 34 zu durchströmen, welche Duftstoff an die Luft abgibt.

Die Baueinheit 30 ist derart am Gehäuse 10, im gezeigten Ausführungsbeispiel am Mantelteil 12 befestigt, dass das Aufnahmefach 32 mit der Einführöffnung 28 fluchtet, sodass die Beduftungskapsel 34 durch die Einführöffnung 28 geradlinig in das Aufnahmefach 32 einschiebbar und nach Gebrauch wieder durch die Einführöffnung 28 dem Aufnahmefach 32 entnehmbar ist.

Die Baueinheit 30 weist ein rahmenartiges aus einem Kunststoff einstückig hergestelltes Trägerelement 38 auf, dessen Aussenquerschnitt wenigstens annähernd dem Innenquerschnitt des Gehäuses 10, im gezeigten Ausführungsbeispiel des Mantelteils 12, entspricht. Dadurch ist die Baueinheit 30 in Längsrichtung L des Gehäuses 10 und Mantelteils 12 in letzteres beziehungsweise letzteren einschiebbar wobei zwischen dem Gehäuse 10 und dem Trägerelement 38 ein Spalt nur geringer Breite verbleiben kann.

Das Trägerelement 38 weist zwei einander gegenüberliegende Seitenwände 40 auf, welche auf der Rückseite durch eine Rückwand 42 miteinander verbunden sind. Die Rückwand 42 weist einen Durchbruch 44 auf, welcher mit seinen Dimensionen der Einführöffnung 28 entspricht und mit dieser fluchtet. Die oberhalb und unterhalb des Durchbruchs 44 verbleibenden Stege 46 der Rückwand 42 weisen in Richtung nach innen abstehende, hülsenförmige Fortsätze 48 auf, in welche zum Befestigen der Baueinheit 30 von der Rückseite her durch entsprechende Löcher im Gehäuse 10 hindurchgeführte Befestigungsschrauben 50 eindrehbar sind.

Die beiden Seitenwände 40 bilden auf ihrer Innenseite je eine Führungsschiene 52 für die Beduftungskapsel 34.

An ihrem obenliegenden Ende sind die beiden Seitenwände 40 mit einem Deckelrahmen 54 miteinander verbunden, welcher in Längsrichtung, einen dem Luftführungszylinder des Ventilators 36 entsprechenden Durchlass 56 aufweist. Zwischen dem oberen der beiden Stege 46 und dem Deckelrahmen 54 ist das Trägerelement 38 auf der Rückseite und daran anschliessend über einem Bereich der Seitenwände 40 offen ausgebildet. Auf der Vorderseite ist das Trägerelement 38 ebenfalls offen ausgebildet, mit der Ausnahme des am oberen Ende die beiden Seitenwände 40 miteinander verbindenden Deckelrahmens 54.

Zwischen dem oberen der Stege 46 und dem Deckelrahmen 54 ist der Ventilator 58 in das Trägerelement 38 eingeschoben und mittels Bolzen 58 am Deckelrahmen 54 und somit Trägerelement 38 befestigt.

An der Vorderseite des Trägerelements 38 ist an diesem ein Schlossträger 60 mittels eines Schnappverschlusses 62 befestigt, welcher ein Fangfach 64 für ein in ihm in und entgegen der Einschubrichtung E der Beduftungskapseln 34 bewegliches Fangschloss 66 aufgenommen ist. Das Fangfach 46 und Fangschloss 46 zusammen bilden ein Schloss 68 für die durch die Einführöffnung 28 in das Aufnahmefach eingeschobene Beduftungskapsel 34. Es hält diese in Arbeitsstellung 34' fest und gibt diese frei wenn auf die Beduftungskapsel 34, beispielsweise wenn sie verbraucht ist, wieder Druck in Einführrichtung E ausgeübt wird. Das Schloss 68 schiebt dann die in Arbeitsstellung 34' mit der Rückseite des Mantelteils 12 fluchtende Beduftungskapsel 34 in Richtung gegen aussen, sodass die Beduftungskapsel 34 mit den Fingern erfasst und aus dem Aufnahmefach 32 entfernt werden kann. Schlösser 68 dieser Art sind allgemein bekannt.

Vom Ventilator 36 verläuft im Luftkanal 24 ein elektrisches Speisekabel 70 zu einer elektronischen Schaltung 72, welche auf einer Printplatte 74 angeordnet ist. Die Printpatte 70 ist an der Bodenplatte 14 mittels eines Schnappverschlusses 76 befestigt.

Die Printplatte 74 trägt einen Schiebeschalter 78, dessen Betätigungselement einen entsprechend an der Rückseite des Mantelteils 12 ausgebildeten Schlitz durchgreift.

Mittels des Schiebeschalters 78 ist der Ventilator 36 ein- und ausschaltbar und weiter ist die Drehzahl einstellbar, wozu in bevorzugter Weise der Schiebeschalter 78 unterschiedliche, beispielsweise 3 Rasterstellungen aufweist. Es ist auch möglich, den Schiebeschalter 78 als Potentiometer auszubinden, um die Drehzahl des Ventilators 36 stufenlos einstellen zu können.

Weiter sind auf der Printplatte 74 mehrere LEDs 80 angeordnet, welche durch den schlitzartigen Lufteinlass 18 durch die Vorderseite des Gehäuses 10 beziehungsweise Mantelteils 12 einsehbar sind und den Betriebszustand des Raumbeduftungsgeräts anzeigen. So beispielsweise EIN/AUS, die gewählte Drehzahl des Ventilators und/oder den Zeitpunkt zum Auswechseln der Beduftungskapsel 34.

In Fig. 2 ist ein mit 81 bezeichneter Mikroschalter gezeigt, welcher an die elektronische Schaltung 72 jeweils ein Signal abgibt, wenn dem Aufnahmefach 32 eine Beduftungskapsel 34 entnommen beziehungsweise wenn eine neue Beduftungskapsel 43 in das Aufnahmefach eingesetzt wird.

Weiter trägt die Printplatte 74 eine Steckdose 84, in welche ein Steckerteil 86 eines Speisegeräts 88 zur elektrischen Speisung der elektronischen Schaltung und des Ventilators 36 durch eine entsprechende Öffnung auf der Rückseite des Gehäuses 10 beziehungsweise Mantelteils 12 einsteckbar ist.

Die Bodenplatte 40 ist im Mantelteil 12 versenkt angeordnet und mittels Spreizstiften in entsprechenden Sacklöchern in den Kantenbereichen des Mantelteils 12 befestigt. Weiter sind an der Bodenplatte 14, auf der Unterseite, Füsse 90 vorzugweise in Form von Gummischeiben befestigt.

Die Deckelplatte 16 ist ebenfalls versenkt im Mantelteil 12 angeordnet und an diesem befestigt, beispielsweise mittels Klebstoff oder ebenfalls mit Spreizstiften.

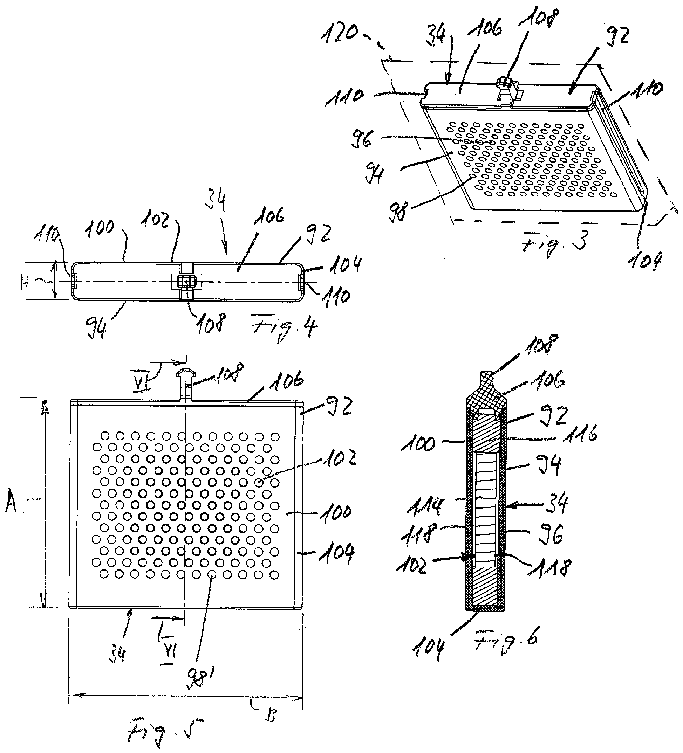

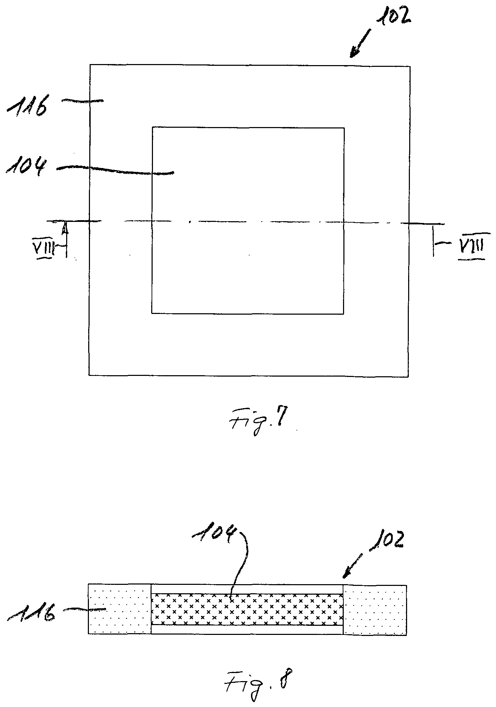

Der Aufbau einer bevorzugten Ausführungsform der Beduftungskapseln 34 geht aus den Fig. 3 bis 8 hervor.

Die Beduftungskapsel 34 weist hier ein quaderförmiges Kapselgehäuse 92, vorzugsweise aus Kunststoff, auf.

Ein erste Wandseite 94, im gezeigten Ausführungsbeispiel die Bodenseite, bildet einen Lufteintritt 96. Dieser ist hier durch eine Vielzahl Durchströmöffnungen 98 gebildet, welche in einem rasterartigen Lochfeld angeordnet sind.

Eine zweite Wandseite 100, hier die Oberseite, welche der ersten Wandseite 94 gegenüberliegt, bildet einen Luftaustritt 102. Letzterer ist durch weitere Durchströmöffnungen 98 gebildet, welche in einem weiterem Lochfeld angeordent sind.

Die erste Wandseite 94 und die zweite Wandseite 100 sind randseitig auf drei Seiten hin durch einen einstückig mit ihnen verbundenen durchgehenden Mantel 104 miteinander verbunden. Auf der vierten Seite - auf der Vorderseite - weist das Kapselgehäuse 92 einen Deckel 106 auf, welcher den Mantel des Kapselgehäuses 92 vollständig verschliesst, sodass einzig der Lufteintritt 96 und der Luftaustritt 102 offen sind.

Der auf der Vorderseite angeordnete Deckel 106 weist mittig eine in Richtung gegen aussen vorstehende Schlosszunge 108 auf, welche dazu bestimmt ist, mit dem Fangschloss 66 zusammenzuwirken.

Der Mantel 104 weist an zwei einander gegenüberliegenden Seiten je eine Führungsnute 110 auf, welche dazu bestimmt ist, beim Einschieben der Beduftungskapsel 34 in das Aufnahmefach 32 mit dessen Führungsschienen 52 zusammenzuwirken.

Weiter sei an dieser Stelle vermerkt, dass die Beduftungskapsel 34, und somit das Kapselgehäuse 92, derart ausgebildet ist, dass sie in Arbeitsstellung 34' einerseits die Einführöffnung 28 verschliesst und andererseits das Durchströmen von Luft, ausser durch den Lufteintritt 96 und Luftaustritt 102, verhindert. Die Beduftungskapsel 34 füllt somit in Arbeitsstellung das Aufnahmefach 32 aus.

Die Höhe H der Beduftungskapsel 34 und somit die Höhe des Mantels 104 ist wesentlich kleiner als die Breite B und Länge A der Beduftungskapsel 34, d.h. im gezeigten Ausführungsbeispiel der ersten Wandseite 94 und zweiten Wandseite 100.

Bevorzugt ist die Höhe H der Beduftungskapsel 34 etwa drei bis sechsmal kleiner als die Länge A beziehungsweise Breite B.

Im vom Kapselgehäuse 92 gebildeten Hohlraum ist ein Duftstoffträger 112 angeordnet. Dieser weist zentrisch einen in der Aufsicht hier rechteckigen Duftträgerbereich 114 auf, welcher von einem Duftstofffreien Randbereich 116 umgeben ist. Der rahmenartige Randbereich 116 ist hier in seinen Abmessungen derart ausgebildet, dass er seitlich wie auch oben und unten am Kapselgehäuse 92 anliegt. Der Duftstoffträgerbereich 114 ist dünner ausgebildet, sodass zwischen der ersten Wandseite 94 sowie der zweiten Wandseite 100 und dem Duftträgerbereich 114 jeweils ein Hohlraum 118 frei bleibt. Dieser ermöglicht ein optimales Durchströmen der Luft durch den Duftstoffträger 112.

Der Duftstoffträger 112 ist porös ausgebildet, wobei bevorzugt die Porosität im Randbereich 116 grösser ist als im Duftträgerbereich 114. Bevorzugt ist der Duftstoffträger 112 aus filzartigem Material hergestellt.

Bevorzugt ist der Duftstoffträgerbereich 114 zusätzlich zu Duftstoff auch mit einem Neutralisationsstoff getränkt.

Weiter dient der Beduftungsträger 112 auch als Filter für Staubpartikel.

Die Beduftungskapseln 34 werden je in einer luftdichten Verpackung 120 geliefert. Es werden Beduftungskapseln 34 unterschiedlicher Geruchsrichtungen zur Verfügung gestellt, sodass der Benutzer den Duft auswählen kann.

Bei der Inbetriebnahme des Raumbeduftungsgeräts wird eine Beduftungskapsel 34 der Verpackung 120 entnommen und mit der Schlosszunge 108 voraus in Einschieberichtung E durch die Einführöffnung 28 in das Aufnahmefach 32 hineingeschoben bis es vom Schloss 68 in Arbeitsstellung 34' fixiert ist. Nachdem das Speisegerät 88 an das elektrische Speisenetz angeschlossen ist, kann durch Betätigen des Schiebeschalters 78 der Ventilator 36 in Betrieb gesetzt werden.

Der Ventilator 36 ist möglichst geräuschlos ausgebildet.

Wünscht der Benutzer einen andern Duftstoff oder ist die Benutzungsdauer einer Beduftungskapsel 34 abgelaufen-beispielsweise kann eine entsprechende Anzeige erfolgen, wie dies weiter oben in der Einleitung erläutert ist - entnimmt der Benutzer die Beduftungskapsel 34 dem Aufnahmefach 32 und ersetzt diese durch eine neue Beduftungskapsel 34.

Selbstverständlich ist es auch möglich, die Beduftungskapseln 34 in einer anderen Form auszugestalten. Beispielsweise kann das Kapselgehäuse 92 kreiszylinderförmig ausgebildet sein. Dies in bevorzugter Weise bei der Verwendung eines Gehäuses 10 mit ebenfalls kreiszylinderförmigem Querschnitt.

Bevorzugt erfolgt die Lieferung des Raumbeduftungsgeräts als Set, zusammen mit mindestens einer Beduftungskapsel 34, bevorzugt mit zwei oder drei Beduftungskapseln 34 wobei alle Beduftungskapseln bevorzugt unterschiedliche Duftstoffe aufweisen.

Es ist auch möglich, dass die Einführöffnung 78 grösser ausgebildet ist und in diese ein entsprechender Vorsprung des Aufnahmefaches 32 beziehungsweise der Baueinheit 30 hineinragt. Dieser Vorsprung weist dann eine Öffnung zum Einführen der Beduftungskapsel 34 mit an diese angepassten Abmessungen auf.

## Patentansprüche

1. Auswechselbare Beduftungskapsel (34) für ein Raumbeduftungsgerät, aufweisend einen Lufteintritt (96), einen Duftstoffträger (112) und einen Luftaustritt (102), wobei die Beduftungskapsel (34) dazu bestimmt ist, im Betrieb sich in einem Aufnahmefach (32) eines Raumbeduftungsgeräts in einer Arbeitsstellung (34') zu befinden und von durch einen Luftkanal (24) strömenden Luft durchströmt zu werden, wobei die Luft durch den Lufteintritt (96), den Duftstoffträger (112) und den Luftaustritt (102)strömt und ein auf dem Duftstoffträger (112) vorhandener Duftstoff dabei die Luft beduftet, **dadurch gekennzeichnet, dass** der Duftstoffträger (112) porös ausgebildet ist und einen mit dem Duftstoff getränkten Duftträgerbereich (114) sowie um diesen herum einen duftstofffreien Randbereich (116) aufweist.

2. Auswechselbare Beduftungskapsel (34) nach Anspruch 1, **gekennzeichnet durch** ein vorzugsweise wenigstens annähernd quaderförmiges Kapselgehäuse (92), wobei eine erste Wandseite (94) des Kapselgehäuses (92) den vorzugsweise **durch** in einem Lochfeld angeordnete Durchströmöffnungen (98) gebildeten Lufteintritt (96) aufweist, eine vorzugsweise der ersten Wandseite (94) gegenüberliegende zweite Wandseite (100) des Kapselgehäuses (92) den vorzugsweise **durch** in einem weiteren Lochfeld angeordnete weitere Durchströmöffnungen (98') gebildeten Luftaustritt (102) aufweist, und im Innern des Kapselgehäuses (92) der Duftstoffträger (112) angeordnet ist.

3. Auswechselbare Beduftungskapsel (34) nach Anspruch 2, **dadurch gekennzeichnet, dass** die erste und die zweite Wandseite (94; 100) einander gegenüberliegen und eine Länge (L) sowie eine Breite (B) aufweisen, welche grösser, vorzugweise wenigstens dreimal so gross ist wie die Höhe (H) des diese Wandseiten (94; 100) dichtend miteinander verbindenden Mantels (104) des Kapselgehäuses (92).

4. Auswechselbare Beduftungskapsel (34) nach einem der Ansprüche 1 bis 3, **dadurch gekennzeichnet, dass** der Duftstoffträger (112) filzartig ausgebildet ist.

5. Auswechselbare Beduftungskapsel (34) nach Anspruch 1, **dadurch gekennzeichnet, dass** der Duftträgerbereich (114) mit dem Duftstoff und mit einem Neutralisationsstoff getränkt ist.

6. Auswechselbare Beduftungskapsel (34) nach Anspruch 1, **dadurch gekennzeichnet, dass** der Randbereich (116) poröser ausgebildet ist als der Duftträgerbereich (114) .

7. Auswechselbare Beduftungskapsel (34) nach einem der Ansprüche 1 bis 6, **gekennzeichnet durch** eine luftdichte Lieferverpackung (120), welche die Beduftungskapsel (34) vollständig umgibt.

8. Set mit einem Raumbeduftungsgerät mit einem einen Lufteinlass (18) für Luft aus einem Raum, einen Luftauslass (20) zum Abgeben von bedufteter Luft in den Raum und eine Einführöffnung (28) aufweisenden Gehäuse (10), einem von Lufteinlass (18) zum Luftauslass (20) führenden Luftkanal (24), einem Ventilator (36) zum Fördern der Luft vom Lufteinlass (18) durch den Luftkanal (24) zum Luftauslass (20), einem Aufnahmefach (32), durch welches hindurch der Luftkanal (24) verläuft und wenigstens einer auswechselbaren Beduftungskapsel (34) nach Anspruch 1, welche durch die Einführöffnung (28) in das Aufnahmefach (32) in die Arbeitsstellung (34') hineinbewegbar ist und welche aus diesem wieder herausbewegbar ist.

## Claims

1. Interchangeable fragrance capsule (34) for a room fragrancing appliance, having an air inlet (96), a fragrance carrier (112) and an air outlet (102), wherein the fragrance capsule (34) is intended to be located in a working position (34') in a receptacle (32) of a room fragrancing appliance during operation and to have air flowing through it through an air duct (24), wherein the air flows through the air inlet (96), the fragrance carrier (112) and the air outlet (102) and a fragrance present on the fragrance carrier (112) fragrances the air in the process, **characterized in that** the fragrance carrier (112) is configured in a porous manner and has a fragrance carrier region (114) impregnated with the fragrance and a fragrance-free peripheral region (116) around it.

2. Interchangeable fragrance capsule (34) according to Claim 1, **characterized by** a preferably at least approximately cuboidal capsule housing (92), wherein a first wall side (94) of the capsule housing (92) has the air inlet (96), which is formed preferably by through-flow openings (98) arranged in a pattern of holes, a second wall side (100), located preferably opposite the first wall side (94), of the capsule housing (92) has the air outlet (102), which is formed preferably by further through-flow openings (98') arranged in a further pattern of holes, and the fragrance carrier (112) is arranged in the interior of the capsule housing (92).

3. Interchangeable fragrance capsule (34) according to Claim 2, **characterized in that** the first and the second wall side (94; 100) are located opposite one another and have a length (L) and a width (B) which is greater, preferably at least three times greater, than the height (H) of the casing (104) of the capsule housing (92), said casing (104) connecting these wall sides (94; 100) together in a sealed manner.

4. Interchangeable fragrance capsule (34) according to one of Claims 1 to 3, **characterized in that** the fragrance carrier (112) is formed in a felt-like manner.

5. Interchangeable fragrance capsule (34) according to Claim 1, **characterized in that** the fragrance carrier region (114) is impregnated with the fragrance and with a neutralizing agent.

6. Interchangeable fragrance capsule (34) according to Claim 1, **characterized in that** the peripheral region (116) is formed in a more porous manner than the fragrance carrier region (114).

7. Interchangeable fragrance capsule (34) according to one of Claims 1 to 6, **characterized by** an airtight delivery pack (120) which entirely surrounds the fragrance capsule (34).

8. Set comprising a room fragrancing appliance having a housing (10) that has an air inlet (18) for air from a room, an air outlet (20) for dispensing fragranced air into the room and an introduction opening (28), having an air duct (24) leading from the air inlet (18) to the air outlet (20), having a fan (36) for delivering the air from the air inlet (18) to the air outlet (20) through the air duct (24), having a receptacle (32) through which the air duct (24) extends, and having at least one interchangeable fragrance capsule (34) according to Claim 1, which is movable into the receptacle (32) into the working position (34') through the introduction opening (28) and which is movable out of said receptacle (32) again.

## Revendications

1. Capsule pour parfumer (34) remplaçable pour un diffuseur de parfum d'ambiance, présentant une entrée d'air (96), un support de parfum (112) et une sortie d'air (102), la capsule pour parfumer (34) étant prévue pour se trouver dans une position de travail (34') dans un compartiment de réception (32) d'un diffuseur de parfum d'ambiance pendant le fonctionnement et pour être parcourue par de l'air s'écoulant à travers un conduit d'air (24), l'air s'écoulant à travers l'entrée d'air (96), le support de parfum (112) et la sortie d'air (102) et un parfum présent sur le support de parfum (112) parfumant ainsi l'air, **caractérisée en ce que** le support de parfum (112) est réalisé de manière poreuse et présente une région de support de parfum (114) imbibée du parfum ainsi qu'une région de bord (116) sans parfum autour de celle-ci.

2. Capsule pour parfumer (34) remplaçable selon la revendication 1, **caractérisée par** un boîtier de capsule de préférence au moins approximativement parallélépipédique (92), un premier côté de paroi (94) du boîtier de capsule (92) présentant l'entrée d'air (96) de préférence formée par des ouvertures de passage (98) disposées dans un champ de perforations, un deuxième côté de paroi (100) du boîtier de capsule (92) de préférence opposé au premier côté de paroi (94) présentant la sortie d'air (102) de préférence formée par des ouvertures de passage supplémentaires (98') disposées dans un champ de perforations supplémentaire, et le support de parfum (112) étant disposé à l'intérieur du boîtier de capsule (92).

3. Capsule pour parfumer (34) remplaçable selon la revendication 2, **caractérisée en ce que** le premier et le deuxième côté de paroi (94; 100) sont opposés l'un à l'autre et présentent une longueur (L) ainsi qu'une largeur (B) qui est supérieure, de préférence au moins trois fois supérieure, à la hauteur (H) de l'enveloppe (104) du boîtier de capsule (92) reliant ces côtés de paroi (94; 100) de manière hermétique l'un à l'autre.

4. Capsule pour parfumer (34) remplaçable selon l'une quelconque des revendications 1 à 3, **caractérisée en ce que** le support de parfum (112) est réalisé à la manière d'un feutre.

5. Capsule pour parfumer (34) remplaçable selon la revendication 1, **caractérisée en ce que** la région du support de parfum (114) est imbibée avec le parfum et avec une substance de neutralisation.

6. Capsule pour parfumer (34) remplaçable selon la revendication 1, **caractérisée en ce que** la région de bord (116) est réalisée de manière plus poreuse que la région du support de parfum (114).

7. Capsule pour parfumer (34) remplaçable selon l'une quelconque des revendications 1 à 6, **caractérisée par** un emballage d'expédition étanche à l'air (120) qui entoure complètement la capsule pour parfumer (34).

8. Ensemble comprenant un diffuseur de parfum d'ambiance avec un boîtier (10) présentant une entrée d'air (18) pour de l'air provenant d'un espace, une sortie d'air (20) pour la diffusion d'air parfumé dans l'espace et une ouverture d'entrée (28), un conduit d'air (24) conduisant de l'entrée d'air (18) à la sortie d'air (20), un ventilateur (36) pour refouler l'air depuis l'entrée d'air (18) à travers le conduit d'air (24) jusqu'à la sortie d'air (20), un compartiment de réception (32) à travers lequel s'étend le conduit d'air (24) et au moins une capsule pour parfumer (34) remplaçable selon la revendication 1, qui peut être introduite à travers l'ouverture d'entrée (28) dans le compartiment (32) dans la position de travail (34') et qui peut à nouveau être ressortie de celui-ci.
